# EUROPÄISCHE PATENTSCHRIFT

(11) **EP 4 333 779 B1**
(45) Veröffentlichungstag und Bekanntmachung des Hinweises auf die Patenterteilung: **29.10.2025**
(21) Anmeldenummer: 22728131.8
(22) Anmeldetag: 05.05.2022
(51) Int. Cl.: A61F 5/02

(54) **RÜCKENSTÜTZGURT**
BACK SUPPORT BELT
CEINTURE DE SOUTIEN DORSAL

(30) Priorität: 07.05.2021 DE 102021204656
(43) Veröffentlichungstag der Anmeldung: 13.03.2024
(73) Patentinhaber: Bauerfeind AG, 07937 Zeulenroda-Triebes (DE)
(72) Erfinder: BAUERFEIND, Hans B., 07937 Zeulenroda-Triebes (DE); STIER, Gerald, 07937 Zeulenroda-Triebes (DE); HEBENSTREIT, Sandro, 07937 Zeulenroda-Triebes (DE)
(74) Vertreter: Wohlfahrt, Jan Günther
(86) Internationale Anmeldenummer: PCT/EP2022/062212
(87) Internationale Veröffentlichungsnummer: WO 2022/234046

(56) Entgegenhaltungen:
- EP-B1- 3 407 842
- WO-A1-2020/011604
- DE-A1- 102013 222 372
- DE-A1- 102017 220 968

## Beschreibung

Die Erfindung betrifft ein Rückenstützgurt-System, besonders eine Rückenorthese oder Reklinationsorthese für die Behandlung und Prävention von Rückenleiden. Das System besitzt einen Beckengurt und zwei davon ausgehende unabhängige linke und rechte Schultergurte, die sich im Bereich des Rückens mehrfach kreuzen.

Rückenstützgurt-Systeme dienen der Aufrichtung des Oberkörpers, beispielsweise zur Behandlung und Korrektur von Haltungsfehlern und verkrümmten Wirbelsäulen, beispielsweise bei Osteoporose. Bekannte Rückenstützgurt-Systeme und Orthesen bestehen im Wesentlichen aus einem Beckengurt, welcher über Becken oder Hüfte eines Patienten, oder allgemein eines Trägers, unter Spannung geschlossen werden kann und welcher dann eine Basis für die Krafteinleitung am Rücken bilden soll. Bekannte Systeme enthalten auch Gurte oder Stützschienen, welche von dem Beckengurt ausgehen und welche die Schultern in räumlichen Bezug zur Wirbelsäule beziehungsweise den oberen Wirbelsäulenabschnitt in Bezug zu dem unteren Wirbelsäulenabschnitt durch gezielte Krafteinleitung repositionieren oder unterstützen sollen. Durch Zusammenwirken dieser Elemente soll die Wirbelsäule des Trägers mechanisch stabilisiert und vor allem im Bereich der Schulter und der oberen Brustwirbelsäule aufgerichtet werden. Je stärker Schultergurte gespannt werden, desto stärker sollen die Schultern nach hinten gezogen werden, wodurch der Oberkörper aufgerichtet werden kann.

Rückenstützgurt-Systeme sollen auch helfen, Belastungen des Rückens beim Heben und Tragen von Lasten zu vermindern, insbesondere übermäßige Belastungen zu vermeiden, indem die dabei vor allem im Bereich der Schultern erzeugten Kräfte gezielt aufgenommen und verteilt werden. Stützgurte sollen die beim Heben und Tragen der Lasten primär über Arme und Schultern auf den Oberkörper wirkende Kräfte unmittelbar in das Becken oder den Rumpf einleiten. Die Wirbelsäule soll so überbrückt und entlastet werden. Daneben soll ein Rückenstützgurtsystem den Tragenden daran erinnern bei übermäßiger Fehlhaltung des Oberkörpers eine schädliche belastende Schonhaltung einzunehmen, und ein Sicherheits- und Stabilisationsgefühl vermitteln. Rückenstützgurt-Systeme sollen damit auch dem Arbeitsschutz bei Gesunden dienen. Gleichzeitig sollen die Rückenstützgurte die Bewegung führen und kontrolliert begrenzen, um für den Rücken ungünstige Fehlhaltungen beim Heben oder Tragen der Lasten zu vermeiden. Dies ist im Arbeitsschutz, in der Rehabilitation und bei bestimmten sportlichen Aktivitäten zweckmäßig. Rückengurt-basierte Orthesen sind aus der JP 2001-062245 A, der JP 2001-224613 A und aus der US 5,499,965 A bekannt.

Nachteilig bei bekannten Rückenstützgurt-Systemen oder Rückenorthesen dieser Art ist der komplexe Aufbau, was das Anlegen für den Träger erschwert und weshalb eine Fehlbenutzung kaum ausgeschlossen werden kann. Die meist starre Ausgestaltung bekannter Bandagen mit Stützelementen und Gurten hilft zwar, eine Stützwirkung zu erreichen, dabei ist aber eine individuelle Anpassung an die spezifische Indikationslage, an das spezifische Therapieerfordernis und/oder an anatomische Gegebenheiten eines Trägers nicht in ausreichendem Maße möglich. Besonders bei Bewegung des Benutzers kommt es in bestimmten Bewegungsphasen, bei besonderer Belastung oder bereits beim Spannen des Rückenstützgurt-Systems während des Anlegens oder danach zu einem ungewollten Verrutschen des Stützgurtsystems am Körper (Migration) in eine Position, die einerseits das Tragegefühl stark beeinträchtigt, zum anderen die Orthese im Wesentlichen wirkungslos machen kann. Vor allem bewegungseingeschränkten Patienten fällt es in der Praxis schwer, derartige Gurtsysteme anzulegen und auch nach dem Anlegen die erforderliche Spannung an den Stützelementen und am Beckengurt zu erzeugen. Ebenso kann es schwerfallen, eine bereits zum Zwecke der ausreichenden Stützwirkung eingerichtete, das heißt vorgespannte, Orthese überhaupt unter dieser Spannung funktionsgerecht anzulegen. Vorgespannte Schultergurte können einen Beckengurt, mit dem sie verbunden sind, bereits beim Anlegen ungünstig nach oben, besonders in den oberen Taillenabschnitt hinein, in Richtung unterer Brustkorb ziehen, sodass ein funktionsgerechter Sitz am Becken nicht mehr erreicht werden kann.

Die DE 10 2018 211 431 A1 offenbart ein Rückenstützgurt. -System gemäß des Oberbegriffs von Anspruch 1.

Der Erfindung lag das technische Problem zugrunde, derartige Rückenstützgurt-Systeme oder Rückenorthesen wie Reklinationsorthesen, die zumindest auf einer Grundstruktur, enthaltend eine Beckengurt-Fassung mit davon ausgehenden krafteinleitenden Schultergurten, basieren, so zu verbessern, dass nicht nur das Anlegen an den Träger unkompliziert ist, sondern bei dem außerdem eine Migration des Systems, besonders der Beckenfassung bereits beim Anlegen oder beim Gebrauch, besonders bei Bewegung des Trägers, wirksam verhindert wird. Dadurch soll die Stütz- und Entlastungswirkung solcher Systeme verbessert und auch über die gesamte Dauer des Tragens aufrechterhalten werden können.

Das technische Problem wird vollständig gelöst durch die neuartige Rückenstützgurt- oder Rückenorthesen-Konstruktion gemäß den Ansprüchen.

Das technische Problem wird insbesondere durch Rückenorthese gelöst, enthaltend:
- einen Beckengurt zum Anlegen an das Becken eines Trägers, mit einem lumbal/sakral positionierbarem Mittelabschnitt und davon jeweils seitlich abgehenden linkem Seitenabschnitt mit linkem Beckengurtende und rechtem Seitenabschnitt mit rechtem Beckengurtende, wobei die Beckengurtenden zum Anlegen miteinander unter Spannung verbindbar sind,
- einen rechten Schultergurt zum Anlegen über die rechte Schulter des Trägers, der über sein erstes Ende mit dem linken Seitenabschnitt des Beckengurts verbunden ist, und
- einen linken Schultergurt zum Anlegen über die linke Schulter des Trägers, der über sein erstes Ende mit dem rechten Seitenabschnitt des Beckengurts verbunden ist,
   wobei sich rechter Schultergurt und linker Schultergurt in einem im angelegten Zustand dorsalen mittigen oberen Kreuzungspunkt, einem im angelegten Zustand dorsalen mittigen unteren Kreuzungspunkt und in zwei im angelegten Zustand jeweils seitlichen Kreuzungspunkten kreuzen, wobei die Rückenorthese ein den Schultergurten zugeordnetes Führungsmodul aufweist, wobei das Führungsmodul umfasst:
   - ein erstes Führungselement,
   - ein zweites Führungselement
   - ein drittes Führungselement
   - ein viertes Führungselement
   - ein erstes Verbindungselement
   - ein zweites Verbindungselement
   - ein drittes Verbindungselement
   - ein viertes Verbindungselement
   - ein fünftes Verbindungselement,
wobei das erste Verbindungselement am dorsalen mittigen oberen Kreuzungspunkt positioniert ist und das erste Führungselement und das zweite Führungselement als Gelenk miteinander verbindet, wobei das zweite Verbindungselement am ersten seitlichen Kreuzungspunkt positioniert ist und das erste Führungselement und das dritte Führungselement als Gelenk miteinander verbindet, wobei das dritte Verbindungselement am zweiten seitlichen Kreuzungspunkt positioniert ist und das zweite Führungselement und das vierte Führungselement als Gelenk miteinander verbindet, wobei sich das dritte Führungselement und das vierte Führungselement im Bereich des dorsalen mittigen unteren Kreuzungspunkts überkreuzen, und wobei der rechte Schultergurt über sein zweites Ende und der linke Schultergurt über sein zweites Ende mit dem Führungsmodul verbunden sind, insbesondere wobei der rechte Schultergurt über sein zweites Ende mit dem vierten Führungselement reversibel verbunden ist und wobei der linke Schultergurt über sein zweites Ende mit dem dritten Führungselement reversibel verbunden ist.

Die vorliegende Erfindung zeichnet sich also insbesondere durch ein Führungsmodul aus verschiedenen Führungselementen und Verbindungselementen aus. Das erste, zweite, dritte und vierte Führungselement bildet über die Verbindungselemente und die Kreuzung des dritten Führungselements und des vierten Führungselements eine Rautenform, wobei die Raute über die gelenkige Lagerung durch die Verbindungselemente und die Verschiebbarkeit des dritten Führungselements und des vierten Führungselements im Kreuzungsbereich in Form und Winkel veränderlich ist. Insbesondere kann sich auch die Höhe, das heißt der Abstand zwischen dem ersten Verbindungselement und dem Kreuzungsbereich des dritten und vierten Führungselements im Bereich des dorsalen, mittigen, unteren Kreuzungspunkts, verändern. Das Führungsmodul umfasst also mindestens ein rautenförmiges Element, das durch die gelenkige Verbindung der Führungselemente in Höhe und Breite variabel ist, also eine Scherenraute darstellt.

Dabei sind die Schultergurte im dorsalen Bereich bevorzugt entlang der Führungselemente geführt, sodass eine Veränderung der Position und/oder Länger der Schultergurte auf die Führungselemente wirkt und zu einer Veränderung der Höhe beziehungsweise Breite des Rautenelements des Führungsmoduls führt. Gleichzeitig führen die Führungselemente bevorzugt die Gurte, sodass in diesem Bereich die Gurtverläufe ebenfalls rautenförmig sind.

Es zeigte sich nun überraschenderweise, dass aufgrund dieser rautenförmigen Gurtverläufe am Rücken des Trägers ein Verrutschen der Orthese besser verhindert wird. Beim Hinsetzen oder Bücken verlängert sich der Rücken und spannt so die Rautenstruktur des Führungsmoduls. Da es sich bei Rauten sozusagen um zwei miteinander verbundene Scheren handelt, kann das Führungsmodul den durch die Bewegung auftretenden Längenänderungen sehr gut folgen, ohne dass das gesamte System an Stabilität einbüßt. Diese Längenänderung kann je nach Bewegung des Trägers eine Verlängerung oder eine Stauchung sein. Die vorliegende Erfindung kann insbesondere ein Hochrutschen der Rückenorthese beim Hinsetzen des Trägers minimieren oder verhindern. Die geometrische Form des Führungsmoduls, also der Raute, ändert sich insbesondere zwischen den Positionen des Trägers "sitzen" und "stehen".

Die vier Führungselemente bilden also die vier Schenkel einer Raute, wobei durch die gelenkige Lagerung in drei Verbindungselementen und die bewegliche Überkreuzung des dritten Führungselements und des vierten Führungselements eine Formänderung, das heißt Längen- und Breitenänderung ermöglicht wird.

Erfindungsgemäß kann das Führungsmodul entweder direkt mit dem dritten Führungselement und dem vierten Führungselement über ein viertes Verbindungselement und ein fünftes Verbindungselement mit dem Beckengurt verbunden sein, oder indirekt über weitere dazwischen liegende Elemente. So können unterhalb der beschriebenen Rautenform des Führungsmoduls durch weitere Führungselemente und Verbindungselemente weitere Rauten oder Halbrauten gebildet werden.

In einer bevorzugten Ausführungsform sind das dritte Führungselement über das vierte Verbindungselement als Gelenk und das vierte Führungselement über das fünftes Verbindungselement als Gelenk direkt mit dem Mittelabschnitt des Beckengurts verbunden.

In dieser Ausführungsform bilden das erste Führungselement, das zweite Führungselement, das dritte Führungselement und das vierte Führungselement eine Raute, wobei die Abschnitte des dritten Führungselements und des vierten Führungselements zwischen dem Bereich, in dem sich diese beiden Führungselemente kreuzen und dem vierten Führungselement und dem fünften Führungselement, mit denen diese beiden Führungselemente mit dem Beckengurt verbunden sind, eine weitere halbe Raute.

In einer alternativen Ausführungsform verbindet das vierte Verbindungselement das dritte Führungselement und ein fünftes Führungselement als Gelenk miteinander, wobei das fünfte Verbindungselement das vierte Führungselement und ein sechstes Führungselement als Gelenk miteinander verbindet, wobei das fünfte Führungselement über ein sechstes Verbindungselement als Gelenk und das sechste Führungselement über ein siebtes Verbindungselement als Gelenk mit dem Mittelabschnitt des Beckengurts verbunden sind.

In einer bevorzugten Ausführungsform sind die zweiten Enden der Schultergurte an den zugeordneten Führungselementen variabel einstellbar oder das vierte und das dritte Führungselement weist jeweils mindestens ein Umlenkelement auf, durch das jeweils der zugeordnete Schultergurt hindurchgeführt ist und das zweite Ende reversibel und variabel am Schultergurt befestigt werden kann.

Unter "Umlenkelement" wird gemäß der Erfindung in erster Linie eine Öse oder Ring, zum Durchführen eines Bands oder Seils, aber auch eine gelagerte Rolle oder ein Rollen-Block verstanden. Eine gleithemmende oder eine gleitverbessernde Beschichtung kann, je nach Anwendungsschwerpunkt vorgesehen sein.

Durch die Umlenkelemente in den Führungselementen können die Schultergurte auf sich selbst festgelegt werden. Dies ermöglicht es mit einer einzigen Orthesenbauform verschiedene Größenbereiche abzudecken.

In einer alternativen Ausführungsform verlaufen die Schultergurte nicht auf dem Führungsmodul, sondern die Schultergurte enden oben am Führungsmodul und sind dort mit diesem befestigt. In dieser Ausführungsform bildet das Führungsmodul ein Zwischenstück am Rücken zwischen Schultergurten und Beckengurt. Somit ist das Führungsmodul beziehungsweise sind die Führungselemente Teil des Gurtverlaufs.

In dieser Ausführungsform kann dann bevorzugt das zweite Ende des linken Schultergurts im Bereich des zweiten Verbindungselements mit dem dritten Führungselement verbunden sein und das zweite Ende des rechten Schultergurts im Bereich des dritten Verbindungselements mit dem vierten Führungselement verbunden sein. In dieser Ausführungsform kann dann bevorzugt das erste Ende des linken Schultergurts im Bereich des ersten Verbindungselements mit dem zweiten Führungselement verbunden sein und das erste Ende des rechten Schultergurts im Bereich des ersten Verbindungselements mit dem ersten Führungselement verbunden sein. In dieser Ausführungsform kann dann bevorzugt das zweite Ende des linken Schultergurts im Bereich des zweiten Verbindungselements mit dem dritten Führungselement verbunden sein, das zweite Ende des rechten Schultergurts im Bereich des dritten Verbindungselements mit dem vierten Führungselement verbunden sein, das erste Ende des linken Schultergurts im Bereich des ersten Verbindungselements mit dem zweiten Führungselement verbunden sein und das erste Ende des rechten Schultergurts im Bereich des ersten Verbindungselements mit dem ersten Führungselement verbunden sein. Die jeweiligen Verbindungen können natürlich reversibel gestaltet sein.

Wenn das erste Ende des linken Schultergurts im Bereich des ersten Verbindungselements mit dem zweiten Führungselement verbunden sein und das erste Ende des rechten Schultergurts im Bereich des ersten Verbindungselements mit dem ersten Führungselement verbunden ist, kann vorgesehen sein, dass ein zusätzlicher Gurt im Bereich des dritten Verbindungselements mit dem zweiten Führungselement verbunden ist und zum rechten Seitenabschnitt des Beckengurtes führt und mit diesem verbunden ist und ein zusätzlicher Gurt im Bereich des zweiten Verbindungselements mit dem ersten Führungselement verbunden ist und zum linken Seitenabschnitt des Beckengurtes führt und mit diesem verbunden ist. Die jeweiligen Verbindungen können natürlich reversibel gestaltet sein.

In einer bevorzugten Ausführungsform sind die Führungselemente aus einem flexiblen und in Zugrichtung im wesentlichen unelastischen Material, beispielsweise Kunststoff. Leder oder Metall, insbesondere Federbandstahl, gebildet.

Die Führungselemente können in einer bevorzugten Ausführungsform aus Federblättern, beispielsweise aus Metall, gebildet sein. Dies hat den Vorteil, dass sich die Führungselemente der Körperform anpassen lassen und in verschiedenen Stärken eingesetzt werden können. Auch lässt sich dadurch eine Vereinheitlichung erreichen, durch die Produktionskosten gespart werden können.

Dem Führungsmodul und insbesondere den Führungselementen können auch zusätzliche Stäbe zugeordnet sein. Diese können dem Verlauf der Führungselemente folgen, insbesondere können sie in der Mitte der Führungselemente deren Längsverlauf folgen. Alternativ können zusätzliche Stäbe auch direkt Verbindungselemente miteinander verbinden, beispielsweise das mittig obere Verbindungselement und das den Beckengurt oder die zwei seitlichen Verbindungselemente miteinander verbinden. Es ist somit möglich die Rückenorthese indikationsbezogen entlang des Verlaufs der Wirbelsäule mit Stäben aufzurüsten. Dies kann für Rückenleiden, insbesondere auch für Skoliose hilfreich sein.

In einer weiteren Ausführung ist das erfindungsgemäße Stützgurt-System zumindest an den Kreuzungspunkten der Schultergurtschlaufen in separaten Kreuzführungen geführt. Dies dient insbesondere der Vereinfachung des Anliegens der Rückenorthese an den Träger oder Benutzer, da die Kreuzführungen die sich kreuzenden Schultergurte an den richtigen Positionen halten. Bevorzugt ist zumindest eine solche Kreuzführung in dem oberen mittigen rückenseitig liegenden Kreuzungspunkt vorgesehen.

Bevorzugt sind die Kreuzführungen in die entsprechenden Verbindungselemente integriert.

Durchlassöffnungen in den Verbindungselementen können beispielsweise solche Kreuzführungen bilden.

In einer bevorzugten Ausführungsform weisen das erste Verbindungselement, das zweite Verbindungselement und/oder das dritte Verbindungselement Durchführungsöffnungen auf, durch die der rechte Schultergurt und der linke Schultergurt verschieblich geführt sind. Bevorzugt weisen das erste Verbindungselement, das zweite Verbindungselement und das dritte Verbindungselement jeweils eine erste Durchführungsöffnung auf, durch die der rechte Schultergurt verschieblich geführt ist und weisen jeweils eine zweite Durchführungsöffnung auf, durch die der linke Schultergurt verschieblich geführt ist.

Die Verbindungselemente verbinden entweder jeweils zwei Führungselemente miteinander oder ein Führungselement mit dem Beckengurt. Dabei sind die beiden Führungselemente oder das Führungselement und der Beckengurt gelenkig miteinander verbunden. Die Verbindungselemente fungieren also nicht nur zur Verbindung, sondern gleichzeitig auch als Scharnier um die gewünschte Beweglichkeit um eine Achse zu gewährleisten. Die Verbindungselemente können beispielsweise als Nieten oder Schrauben ausgeführt sein, die durch Löcher der Führungselemente oder durch das Material des Beckengurts hindurch reichen. Als entsprechende Löcher in den Führungselementen können bei Bedarf auch Langlöcher vorgesehen sein.

Langlöcher bieten sich insbesondere an, wenn unterhalb der beschriebenen Rautenform des Führungsmoduls durch weitere Führungselemente und Verbindungselemente weitere Rauten oder Halbrauten gebildet werden. Dies ermöglich eine höhere Bewegungsfreiheit, die beispielsweise bei einer Skoliose des Trägers vorteilhaft ist.

Die Verbindungselemente, insbesondere die Verbindungselemente, die zwei Führungselemente gelenkig miteinander verbinden, können auch als Festkörpergelenke ausgestaltet sein. Sie können beispielsweise aus einem biegsameren Material als die Führungsgelenke hergestellt sein. In einer solchen Ausgestaltung kann das Führungsmodul einstückig ausgebildet sein. Beispielsweise können die einzelnen Bauteile des Führungsmoduls über flexible / elastische Verbindungen materiell verbunden sein, so dass das Führungsmodul ein Zweikomponentenelement ist. Zusätzliche Schlitze in den Verbindungselementen oder im einstückigen Führungsmodul können die Bewegungsneigung des Materials verstärken. Die Schlitze können beispielsweise Mäanderförmig sein, oder andere Geometrien aufweisen.

Dem Fachmann sind geeignete Ausführungsformen für solche Verbindungselemente, die die gewünschte Beweglichkeit in einer Achse ermöglichen, bekannt.

In einer bevorzugten Ausführungsform ist das vierte Verbindungselement und das fünfte Verbindungselement jeweils ein gelenkig verbundener Krafteinleitungsgurt befestigt, wobei die Krafteinleitungsgurte reversibel mit dem Beckengurt verbunden sind. Dies bietet sich insbesondere in der Ausführungsform der Rückenorthese an, in der das dritte Führungselement und das vierte Führungselement nicht direkt mit dem Beckengurt verbunden sind.

Dem Fachmann sind geeignete Ausführungsformen und Materialien der Schultergurte bekannt, beispielsweise aus der DE 10 2018 211 431 A1. Die Schultergurte können alternativ auch als Seilzugsystem oder Bowdenzug ausgebildet sein.

Die Schultergurte können beispielsweise elastisch, unelastisch, teilelastisch, oder flexibel aber unelastisch ausgeführt sein.

Eine Spannung der Schultergurte kann durch eine variable und flexible Fixierung der Gurte auf den Beckengurt und/oder auf den Führungselementen erfolgen. Alternativ, beispielsweise bei einer Verwendung eines Bowdenzug- oder Seilzugsystems, können auch Spannelemente vorgesehen sein, beispielsweise Spannknöpfe zum Aufwickeln des Spannseils.

Auch kann vorgesehen sein, dass die Schultergurte jeweils zwei geteilt sind und somit einen ersten Teilabschnitt und einen zweiten Teilabschnitt aufweisen, wobei die beiden Teilabschnitte über ein Umlenkelement miteinander verbunden sind. Dabei kann ein Teilabschnitt fest mit dem Umlenkelement verbunden sein, der andere Gurtabschnitt durch das Umlenkelement hindurchgeführt sein und dann beispielsweise über einen Klettverschluss mit sich selber reversibel verbunden sein. Dies kann das Spannen der Schultergurte durch den Träger erleichtern.

In einer bevorzugten Ausführungsform sind die ersten Enden der Schultergurte reversibel, insbesondere über einen Klettverschluss mit dem Beckengurt verbunden.

Beispielsweise können der linke Seitenabschnitt und der rechte Seitenabschnitt des Beckengurts Festlegebereiche aufweisen, auf denen die ersten Enden der Schultergurte reversibel und frei positionierbar anklettbar sind. Dadurch sind eine Grundstraffung und Positionierung der Schultergurte möglich.

Der Beckengurt selbst kann in herkömmlicherweise Weise, beispielsweise durch einen Klettverschluss an den beiden Beckengurtenden geschlossen werden, insbesondere unter Spannung geschlossen werden. Entsprechende Ausgestaltungsmöglichkeiten eines Beckengurts sind dem Fachmann bekannt.

In bevorzugten Ausführungen der Erfindung ist mindestens ein Bereich der Schultergurte jeweils als besonders elastischer Gurtabschnitt ausgebildet.

Unter "elastischer Gurtabschnitt" wird gemäß der Erfindung in erster Linie ein elastisches, das heißt durch Zugbeanspruchung dehnbares, flexibles Band, besonders in Form eines gewebten Gurtes verstanden. Funktionsäquivalente Ausführungen besonders Elastomerbänder, bevorzugt aus Gummi, Silikonkautschuk, Polyurethan und ähnlichen, sind damit ebenso erfasst. Alternativ sind darunter auch elastische Spannseile mit Einlagen aus Elastomerfasern zu verstehen. Es ist vorgesehen, dass diese elastischen Gurtabschnitte, besonders im Vergleich zu den übrigen Abschnitten der Schultergurte, mehr elastisch, das heißt bei Zugbeanspruchung weiter dehnbar, sind. In einer bevorzugten Variante sind diese elastischen Gurtabschnitte jeweils die einzigen Bestandteile der Schultergurte, die unter den Zugkräften, die bei sachgemäßer Benutzung der Orthese auftreten können, besonders beim Beugen des Rückens, gedehnt, das heißt verlängert, werden.

Dieser elastische Gurtabschnitt ist bevorzugt jeweils an demjenigen Bereich der Schultergurte angeordnet, der jeweils über den hinteren oberen Rücken zu den Schultern nach oben zieht. Da gerade in diesem Körperbereich die Längenänderung beim Beugen des Rückens besonders stark ausgeprägt ist, kann der dort angeordnete besonders elastische Gurtabschnitt einen übermäßigen Zug der Gurte verringern, das heißt besonders durch seine Eigendehnung beim Beugen kompensieren. So dient der elastische Gurtabschnitt durch seine Dehnbarkeit einerseits der Zugkraftbegrenzung des von den Schultergurten in den Beckengurt eingeleiteten Zugs, was die Migration des Beckengurts weiter verhindert. Zum anderen kann damit auch die Längenveränderung des Rückens beim Beugen kompensiert werden, was die Schultergurte an der Schulter in Position hält, Bewegungsfreiheit gibt und den Tragekomfort und die Wirksamkeit des Rückengurt-Systems erhöht.

In einer Variante davon ist dieser elastische Endabschnitt zusätzlich mit einer separat ausgebildeten und parallel zu dem elastischen Abschnitt geführten mechanischen Dehnungsbegrenzung versehen. Die Dehnungsbegrenzung kann als mindestens ein weniger elastisches oder unelastisches Band ausgebildet sein, das mit jeweils den Enden des elastischen Abschnitts verbunden ist. Dies kann, je nach gewünschtem Therapieziel oder Anwendungszweck des Systems, der kontrollierten Bewegungsführung bei der Beugung dienen. Insbesondere soll eine übermäßige Beugung des Rückens verhindert werden, was therapeutisch, beispielsweise in der postoperativen Rehabilitation oder im Bereich des Arbeitsschutzes beim Heben von Lasten zur Reklination und Haltungsverbesserung und der daraus resultierenden Verbesserung der Lasteinleitung im Rücken, angezeigt sein kann.

In einer bevorzugten Ausführungsform ist jeweils zwischen dem ersten Ende des Schultergurts und dem seitlichen Kreuzungspunkt ein elastischer Gurtabschnitt ausgebildet, dessen Elastizität gleich oder höher ist als diejenige des Schultergurts selbst.

Das erfindungsgemäße Rückenstützgurt-System erlaubt, den Träger daran zu erinnern, bei einer Fehlhaltung des Oberkörpers, das heißt besonders: Rumpf stark nach vorn gebeugt und im schlimmsten Fall zusätzliche Rotation des Rückens in der vorgebeugten Haltung, eine schonendere Haltung einzunehmen, das heißt besonders: eine aufrechte Haltung. Beim Heben von Lasten mit gekrümmter Wirbelsäule resultiert Lastverschiebung auf den vorderen Teil des ventralen Pfeilers des Wirbelkörpers, durch Aufrichtung der Wirbelsäule und Heben aus den Knien (aufrechte Haltung) verteilt sich die Last vorteilhafterweise flächig auf den ventralen Pfeiler. Die aufrechte Haltung kann vom Tragenden besonders dann eingenommen, wenn er bei Beugung des Oberkörpers nach vorn einen verstärkten Zug über das erfindungsgemäße Schultergurtsystem erfährt. Der Beckengurt dient dabei als "Anker" und legt den zentralen Fixpunkt fest, der dazu dient, aus der Bewegung eine Kraft an den Schultern in dorsaler Richtung über das Gurtsystem zu erzeugen. Ein weiterer Nebeneffekt des straff anliegenden gespannten Beckengurtes des erfindungsgemäßen Rückenstützgurt-Systems ist die Vermittlung eines Sicherheits- und Stabilisationsgefühls an den Träger des Systems.

In einer besonderen Ausführung ist das erfindungsgemäße Rückenstützgurt-System daher auf einer elastischen Weste oder Jacke aufgerüstet, wobei die Schultergurte und/oder der Beckengurt zumindest abschnittsweise an der Weste oder Jacke fixiert sind.

Die vorliegende Erfindung löst das ihr zugrunde liegende technische Problem auch durch ein Bekleidungsstück, enthaltend eine dehnbare Weste oder Jacke und ein erfindungsgemäßes Rückenstutzgurtsystem, wobei Schultergurte und Beckengurt zumindest abschnittsweise auf einer Weste oder Jacke aufgerüstet und mit dieser fest verbunden sind. Das erfindungsgemäße Bekleidungsstück kann beispielsweise eine Arbeitsschutzkleidung sein.

Im einfachsten Falle ist das Rückenstützgurtsystem mit der Weste oder Jacke vernäht. In einer alternativen Variante sind die Stützgurte an der Weste oder Jacke in Laschen geführt. Die Ausbildung an einer Weste oder Jacke erlaubt ein vereinfachtes An- und Ablegen, was bei allem bei kurzfristiger und wiederkehrender Verwendung des Rückenstützgurt-Systems durch einen Träger, beispielsweise im Bereich des Arbeitsschutzes, sehr nützlich ist. Die verwendete Weste oder Jacke besteht aus elastischem Gewebe oder Gestrick. Alternativ können flexible Westen, wie sie beispielsweise als Warnwesten an sich bekannt sind, mit dem erfindungsgemäßen Rückenstützgurt-System aufgerüstet werden.

Unter einem "Träger" wird vorliegend eine Person verstanden, die beispielsweise im Rahmen von Arbeitsschutzmaßnahmen das erfindungsgemäße Gurtsystem anlegt und benutzt, das heißt trägt, um damit einer übermäßigen Belastung oder Fehlhaltung beispielsweise beim Arbeiten, besonders beim Tragen von Lasten, vorzubeugen. Weiter wird darunter ein Patient verstanden, der beispielsweise im Rahmen von Therapiemaßnahmen das erfindungsgemäße Gurtsystem anlegt und benutzt, das heißt trägt, um damit eine übermäßige Belastung oder Fehlhaltung eines vorgeschädigten, erkrankten Bewegungsapparats, entgegenzuwirken oder eine gezielte Bewegungsführung im Sinne einer Therapie des erkrankten Bewegungsapparats zu ermöglichen.

Die vorliegende Erfindung löst das ihr zugrunde liegende technische Problem auch durch die Verwendung einer erfindungsgemäßen Rückenorthese oder eines erfindungsgemäßen Bekleidungsstücks zum Schutz vor nachteiligen Belastungen des Rückens.

Insbesondere ist dabei eine nichtmedizinische Verwendung umfasst, bei der die erfindungsgemäße Rückenorthese als Schutz, insbesondere als Arbeitsschutz getragen wird, also insbesondere prophylaktisch getragen wird.

Die Erfindung wird durch die nachfolgenden spezifischen Ausführungsbeispiele näher illustriert, ohne dass diese beschränkend zu verstehen wären.

### BEZUGSZEICHENLISTE

Rückenorthese (10)
Rückenorthese (11)
rechter Schultergurt (22)
erstes Teilstück des rechten Schultergurts (22a)
zweites Teilstück des rechten Schultergurts (22b)
zusätzlicher rechter Gurt (22c)
zweites Ende des rechten Schultergurts (23)
linker Schultergurt (24)
erstes Teilstück des linken Schultergurts (24a)
zweites Teilstück des linken Schultergurts (24b)
zusätzlicher linker Gurt (24c)
zweites Ende des linken Schultergurts (25)
erstes Ende des rechten Schultergurts (26)
erstes Ende des linken Schultergurts (27)
Umlenkelement am rechten Schultergurt (28)
Umlenkelement am linken Schultergurt (29)
Beckengurt (30)
Mittelabschnitt (31)
linker Seitenabschnitt (32)
linkes Beckengurtende (33)
rechter Seitenabschnitt (34)
rechtes Beckengurtende (35)
Klettelement (36a)
Klettelement (36b)
Klettverschluss (36)
mittiger oberer Kreuzungspunkt (41)
linker seitlicher Kreuzungspunkt (42)
mittiger unterer Kreuzungspunkt (43)
rechter seitlicher Kreuzungspunkt (44)
elastischer Gurtabschnitt des rechten Schultergurts (86)
elastischer Gurtabschnitt des linken Schultergurts (87)
Führungsmodul (100)
erstes Führungselement (101)
zweites Führungselement (102)
drittes Führungselement (103)
viertes Führungselement (104)
fünftes Führungselement (105)
sechstes Führungselement (106)
erstes Verbindungselement (141)
zweites Verbindungselement (142)
drittes Verbindungselement (143)
viertes Verbindungselement (144)
fünftes Verbindungselement (145)
sechstes Verbindungselement (146)
siebtes Verbindungselement (147)
Durchführungsöffnung des ersten Verbindungselements (151)
erste Durchführungsöffnung des ersten Verbindungselements (151a)
zweite Durchführungsöffnung des ersten Verbindungselements (151b)
Durchführungsöffnung des zweiten Verbindungselements (152)
erste Durchführungsöffnung des zweiten Verbindungselements (152a)
zweite Durchführungsöffnung des zweiten Verbindungselements (152b)
Durchführungsöffnung des dritten Verbindungselements (153)
erste Durchführungsöffnung des dritten Verbindungselements (153a)
zweite Durchführungsöffnung des dritten Verbindungselements (153b)
Krafteinleitungsgurt am vierten Verbindungselement (154)
Krafteinleitungsgurt am fünften Verbindungelement (155)
Umlenkelement des dritten Führungselements (163)
Umlenkelement des vierten Führungselements (164)
Jacke (200)

- **Figur1**: zeigt eine erste Ausführungsform der erfindungsgemäßen Rückenorthese in zwei verschiedenen Positionen.
- **Figur 2**: zeigt die Rückenorthese von Figur 1 bei einer stehenden Person.
- **Figur 3**: zeigt die Rückenorthese von Figur 1 bei einer sitzenden Person.
- **Figur 4**: zeigt die Rückansicht der Rückenorthese von Figur 1.
- **Figur 5**: zeigt die Seitenansicht der Rückenorthese von Figur 1.
- **Figur 6**: zeigt die Vorderansicht der Rückenorthese von Figur 1.
- **Figur 7**: zeigt das Führungsmodul der Rückenorthese von Figur 1.
- **Figur 8**: zeigt schematisch den Verlauf des Führungsmoduls in der Rückenorthese von Figur 1.
- **Figur 9**: zeigt eine zweite Ausführungsform der erfindungsgemäßen Rückenorthese.
- **Figur 10**: zeigt die Rückansicht der Rückenorthese von Figur 9.
- **Figur 11**: zeigt die Seitenansicht der Rückenorthese von Figur 9.
- **Figur 12**: zeigt einen Teil der Rückenorthese von Figur 9 in schräg vorderer Ansicht.
- **Figur 13**: zeigt die Rückansicht der Rückenorthese von Figur 9 beim Spannen der Schultergurte.
- **Figur 14**: zeigt eine Jacke/Weste mit einer erfindungsgemäßen Rückenorthese.
- **Figur 15**: zeigt das Grundprinzip einer alternativen Ausführungsform der erfindungsgemäßen Rückenorthese.

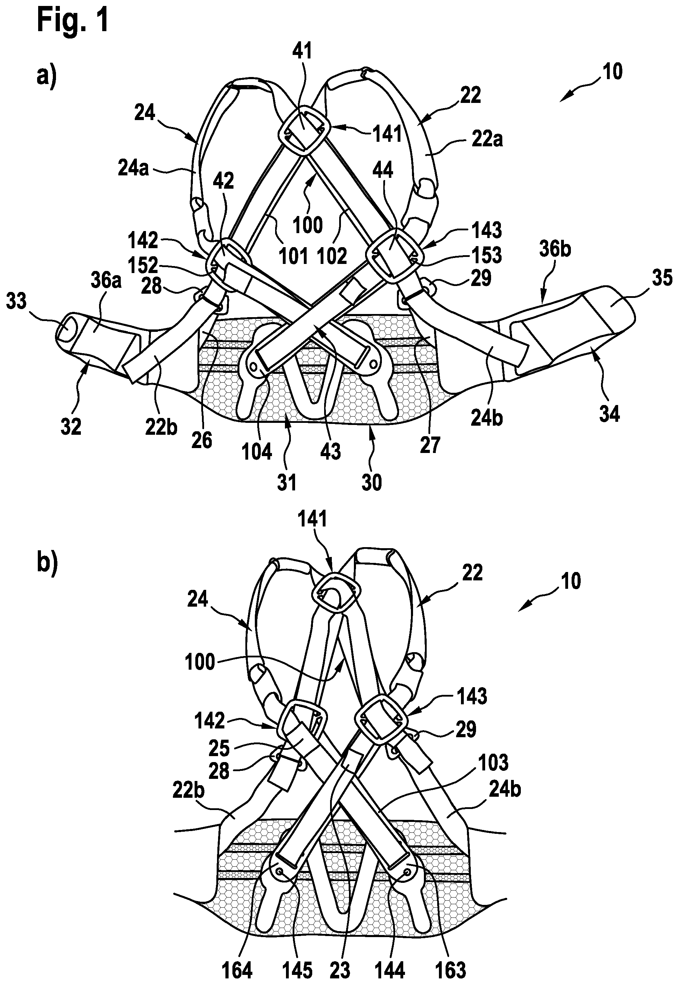

Figur 1 zeigt eine erste Ausführungsform der erfindungsgemäßen Rückenorthese (10). In den Figuren 1a und 1b ist die Rückenorthese (10) in verschiedenen Positionen gezeigt. Die Rückenorthese (10) umfasst einen Beckengurt (30), einen rechten Schultergurt (22), der im getragenen Zustand die rechte Schulter umfasst, einen linken Schultergurt (24), der im getragenen Zustand die linke Schulter des Trägers umfasst, und ein unter den Schultergurten liegendes Führungsmodul (100).

Der Beckengurt (30) weist einen Mittelabschnitt (31), einen linken Seitenabschnitt (32) mit einem linken Beckengurtende (33) und einem rechten Seitenabschnitt (34) mit einem rechten Beckengurtende (35) auf. Der linke Seitenabschnitt (32) und der rechte Seitenabschnitt (34) können über Klettelemente (36a, 36b) eines Klettverschlusses miteinander verbunden werden. Der rechte Schultergurt (22) ist über sein erstes Ende (26) mit dem linken Seitenabschnitt (32) des Beckengurts (30) verbunden und der linke Schultergurt (24) ist über sein erstes Ende (27) mit dem rechten Seitenabschnitt (34) des Beckengurts (30) verbunden. In der vorliegenden Ausführungsform sind die beiden Schultergurte (22, 24) jeweils zweiteilig (22a, 22b, 24a, 24b) ausgeführt, wobei die beiden Teile (22a, 22b, 24a, 24b) der Schultergurte (22, 24) jeweils über Umlenkösen (28, 29) miteinander verbunden sind, sodass eine Längenverstellbarkeit gegeben ist, wobei die am Beckengurt (30) befestigten Teilstücke (22b, 24b) nach der Umlenkung zur Fixierung an sich selbst festgeklettet werden können.

Der rechte Schultergurt (22) und der linke Schultergurt (24) kreuzen sich insgesamt vier Mal, nämlich an einem mittigen oberen Kreuzungspunkt (41), an einem linken seitlichen Kreuzungspunkt (42), an einem mittigen unteren Kreuzungspunkt (43) und an einem rechten seitlichen Kreuzungspunkt (44). Im Bereich zwischen diesen Kreuzungspunkten (41, 42, 43, 44) und zusätzlich bis zum Mittelabschnitt (31) des Beckengurts (30) ist das Führungsmodul (100) mit seinen verschiedenen Elementen angesiedelt. Am mittig oberen Kreuzungspunkt (41) ist das erste Verbindungselement (141) des Moduls (100) zu sehen. Das zweite Verbindungselement (142) befindet sich am linken seitlichen Kreuzungspunkt (42) und das dritte Verbindungselement (143) befindet sich am rechten seitlichen Kreuzungspunkt (44). Zu sehen sind von diesen drei Verbindungselementen (141, 142, 143) primär die Durchführungsöffnungen (151, 152, 153) für die Schultergurte (22, 24). Zu sehen sind unter den Gurten teilweise auch das erste Führungselement (101), das zweite Führungselement (102), das dritte Führungselement (103) und das vierte Führungselement (104). Das dritte Führungselement (103) ist über ein viertes Verbindungselement (144) und das vierte Führungselement (104) ist über ein fünftes Verbindungselement (145) beweglich am Mittelabschnitt (31) des Beckengurts (30) befestigt. Das dritte Führungselement (103) und das vierte Führungselement (104) weisen jeweils ein Umlenkelement (163, 164) auf, durch die jeweils das zweite Ende (23) des rechten Schultergurts (22) und das zweite Ende (25) des linken Schultergurts (24) hindurchgeführt sind, sodass jeweils das zweite Ende (23, 25) über einen Klettverschluss reversibel und variabel an den Schultergurten (22, 24) befestigt werden kann, sodass auch hier eine Spannmöglichkeit der Schultergurte (22, 24) vorhanden ist.

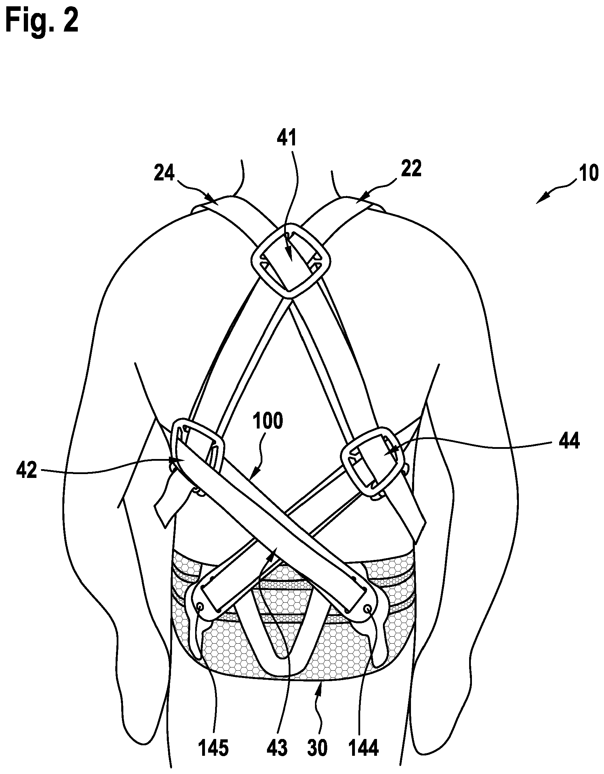

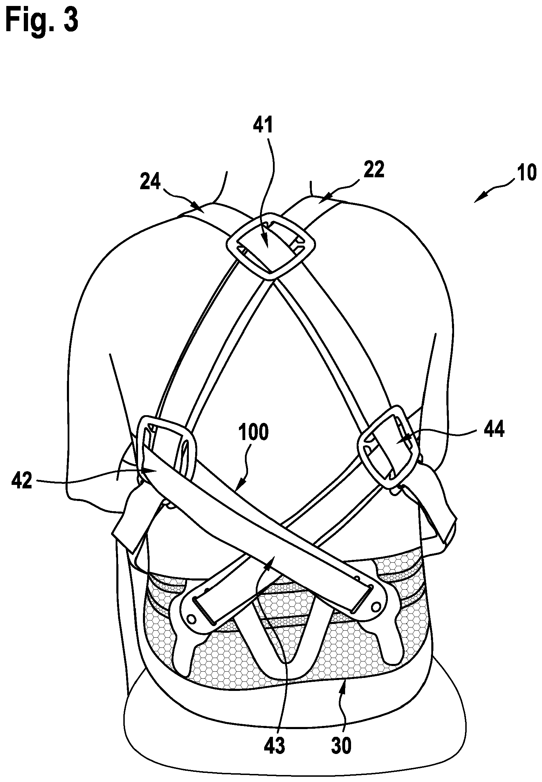

Figur 2 zeigt die Rückenorthese (10) an einem stehenden Träger, Figur 3 zeigt dieselbe Rückenorthese (10) an einem sitzenden Träger. Deutlich zu erkennen ist die durch den rechten Schultergurt (22) und den linken Schultergurt (24) durch das Führungsmodul (100) gebildete Rautenform zwischen den vier Kreuzungspunkten (41, 42, 43, 44). Ebenfalls zu erkennen ist eine darunter liegende halbe Rautenform, die durch den mittig unteren Kreuzungspunkt (43), an dem sich die beiden Schultergurte (22, 24) kreuzen und dem vierten Verbindungselement (144) und dem fünften Verbindungselement (145) gebildet wird.

Vergleicht man die Rautenform der oberen geschlossenen Raute und der darunterliegenden halben Raute, so ist zu erkennen, dass die Schenkellänge der oberen Raute sich beim Hinsetzen des Trägers verlängert, während sich die Schenkellänge der unteren offenen halben Raute verkürzt. Dabei wird der Beckengurt (30) nicht nach oben verschoben und bleibt somit zur optimalen indikationsgerechten Behandlung positionstreu.

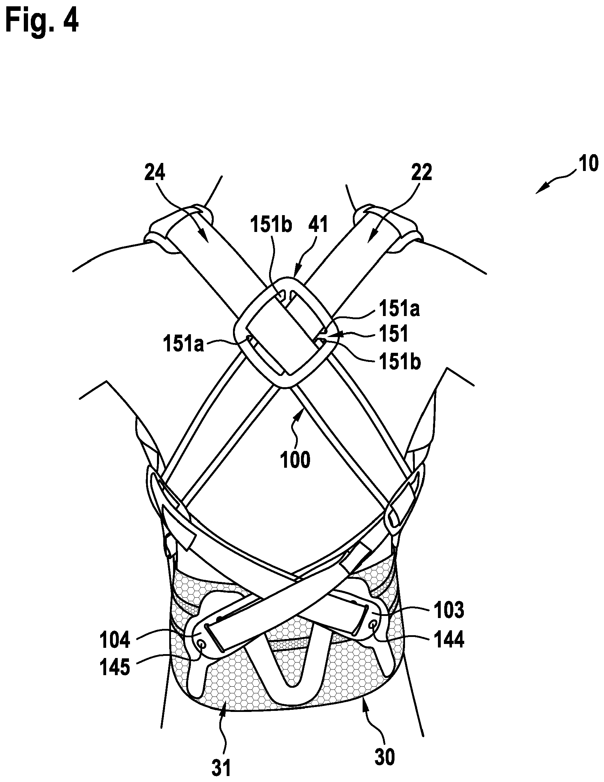

Figur 4 zeigt die Rückansicht der Rückenorthese (10) in der Ausführungsform nach Figur 1. Unter den beiden Schultergurten (22, 24) befindet sich das Führungsmodul (100), das aus den hier nicht sichtbaren Teilen erstes Führungselement, zweites Führungselement, erstes Verbindungselement, zweites Verbindungselement und drittes Verbindungselement sowie aus den zumindest teilweise sichtbaren Teilen drittes Führungselement (103), viertes Führungselement (104), viertes Verbindungselement (144) und fünftes Verbindungselement (145) gebildet wird. Die beiden unteren Führungselemente (103, 104) sind über die beiden unteren Verbindungselemente (144, 145) drehbar an dem Mittelabschnitt (31) des Beckengurts (30) befestigt. Am ersten Verbindungselement im mittigen oberen Kreuzungspunkt (41) der beiden Schultergurte (22, 24) ist ein Durchführungselement (151) angebracht, das eine erste Durchführungsöffnung (151a) für den rechten Schultergurt (22) und eine zweite Durchführungsöffnung (151b) für den linken Schultergurt (24) aufweist, sodass die beiden Schultergurte (22, 24) im Kreuzungspunkt (41) geführt sind aber gegeneinander gleiten können, was eine Längen- und Winkelveränderung dieser Gurtabschnitte zueinander ermöglicht.

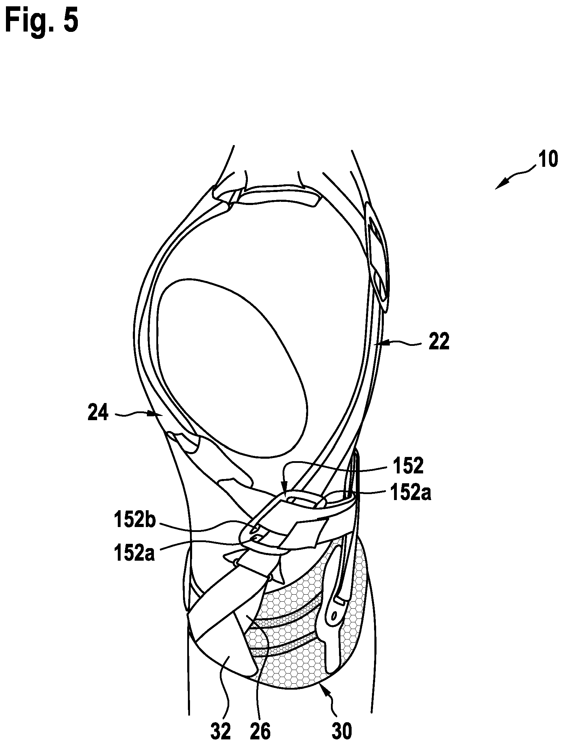

Figur 5 zeigt die Seitenansicht der Rückenorthese (10) von Figur 4. Am nicht sichtbaren zweiten Verbindungselement ist wieder ein Durchführungselement (152) mit einer ersten Durchführungsöffnung (152a) für den rechten Schultergurt (22) und einer zweite Durchführungsöffnung (152b) für den linken Schultergurt (24) zu sehen. Das erste Ende (26) des rechten Schultergurts (22) ist mit dem linken Seitenabschnitt (32) des Beckengurts (30) über eine Klettverbindung verbunden.

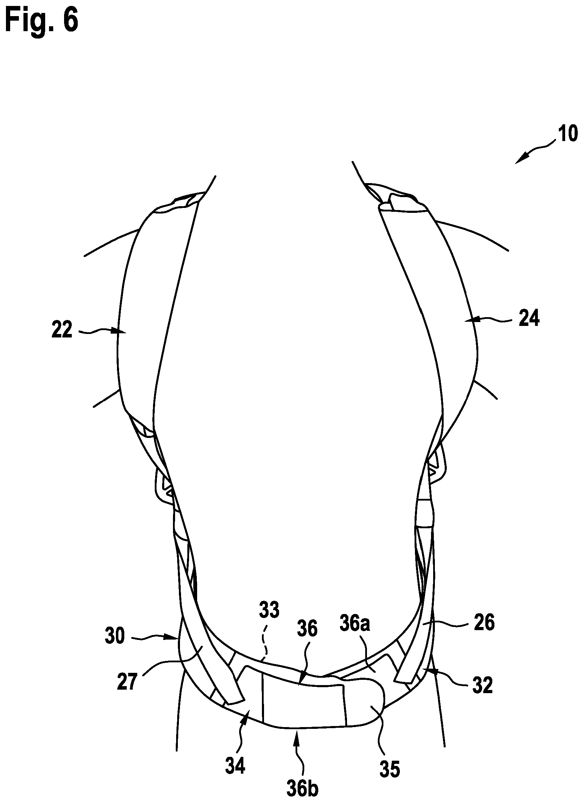

Figur 6 zeigt die Rückenorthese (10) der Figuren 4 und 5 in Vorderansicht. Zu sehen ist der Verlauf des rechten Schultergurts (22) um die rechte Schulter und des linken Schultergurts (24) um die linke Schulter des Trägers. Der linke Seitenabschnitt (32) mit dem linken Beckengurtende (33) und der rechte Seitenabschnitt (34) mit dem rechten Beckengurtende (35) des Beckengurts (30) sind über einen Klettverschluss (36) aus einem ersten Klettelement (36a) am linken Seitenabschnitt (32) und einem auf der Innenseite befindlichen zweiten Klettelement (36b) am rechten Seitenabschnitt (34) zur Straffung des Beckengurts (30) miteinander reversibel verbunden. Das erste Ende (27) des linken Schultergurts (24) ist auf dem rechten Seitenabschnitt (34) des Beckengurts (30) reversibel und verstellbar festgeklettet, das erste Ende (26) des rechten Schultergurts (22) ist auf dem linken Seitenabschnitt (32) reversibel und verstellbar festgeklettet.

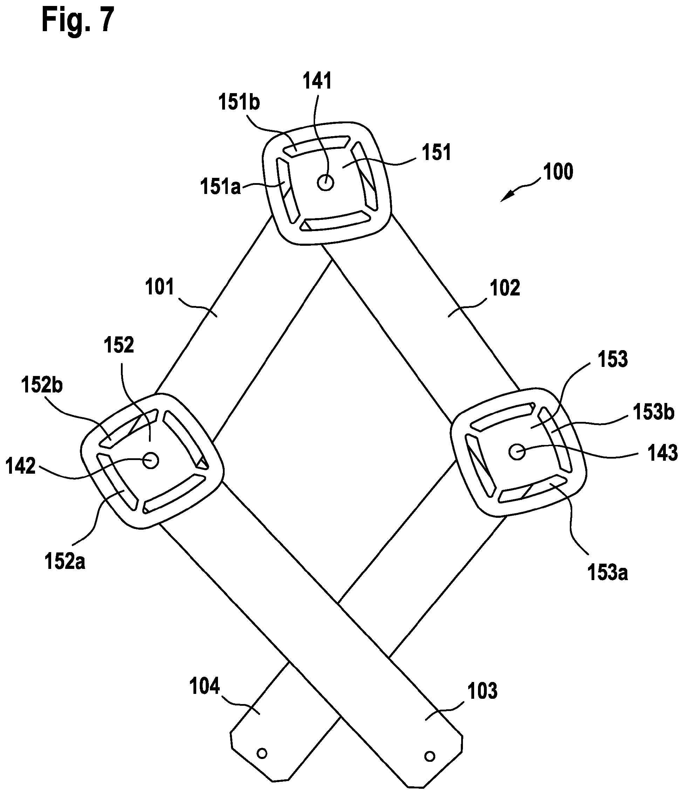

Figur 7 zeigt das Führungsmodul (100) der Rückenorthese aus den Figuren 1 bis 6. Das Führungsmodul (100) umfasst ein erstes Führungselement (101) und ein zweites Führungselement (102), die über ein als Niete ausgeführtes erstes Verbindungselement (141) drehbar miteinander verbunden sind. Das erste Führungselement (101) ist über das zweite Verbindungselement (142) drehbar mit dem dritten Führungselement (103) verbunden, das zweite Führungselement (102) ist über ein drittes Verbindungselement (143) drehbar mit dem vierten Führungselement (104) verbunden. Das dritte Führungselement (103) und das vierte Führungselement (104) kreuzen sich im unteren Bereich und haben am unteren Ende Löcher, durch die ein nicht gezeigtes viertes Verbindungselement und ein nicht gezeigtes fünftes Verbindungselement als Nieten hindurchführen können und somit das dritte Führungselement (103) und das vierte Führungselement (104) beweglich mit dem Beckengurt verbinden. Die drei Verbindungselemente (141, 142, 143) weisen jeweils Durchführungselemente (151, 152, 153) auf. Diese Durchführungsöffnungselemente (151, 152, 153) haben jeweils eine erste Durchführungsöffnung (151a, 152a, 153a) und eine zweite Durchführungsöffnung (151b, 152b, 153b), durch die die Schultergurte der Rückenorthese verschieblich geführt werden können, sodass der mittig obere Kreuzungspunkt, der linke seitliche Kreuzungspunkt und der mittig untere Kreuzungspunkt durch die drei Verbindungselemente (141, 142, 143) definiert und positioniert werden.

Die Führungselemente (101, 102, 103, 104) sind aus einem flexiblen, aber in Zugrichtung im wesentlichen unelastischen Kunststoff gebildet, können aber auch aus Metall, beispielsweise einem Federbandstahl, gebildet werden.

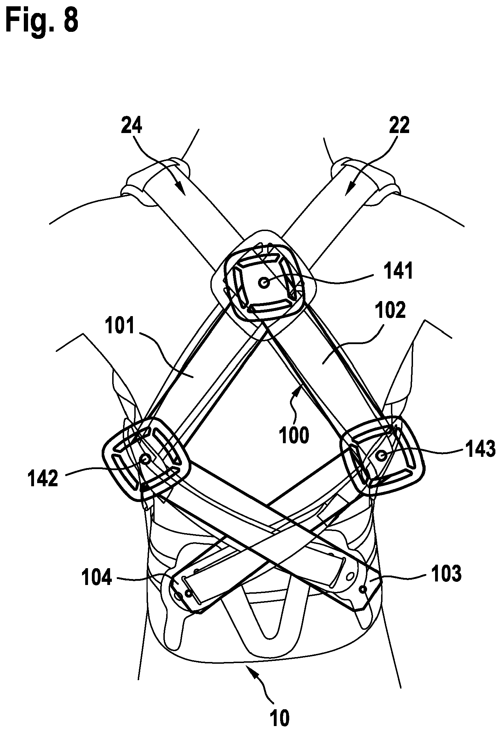

In Figur 8 ist schematisch die Positionierung des Führungsmoduls (100) am ventralen Bereich der Rückenorthese (10) gezeigt. Dadurch ist zu erkennen, dass der rechte Schultergurt (22) dem Verlauf des ersten Führungselements (101) und des vierten Führungselements (104) folgt, und der linke Schultergurt (24) dem Verlauf des zweiten Führungselements (102) und des dritten Führungselements (103) folgt. Weiterhin ist zu erkennen, dass das erste Verbindungselement (141) im Bereich des mittigen oberen Kreuzungspunkts liegt, das zweite Verbindungselement (142) im Bereich des linken seitlichen Kreuzungspunkts und das dritte Verbindungselement (143) im Bereich des rechten seitlichen Kreuzungspunkts.

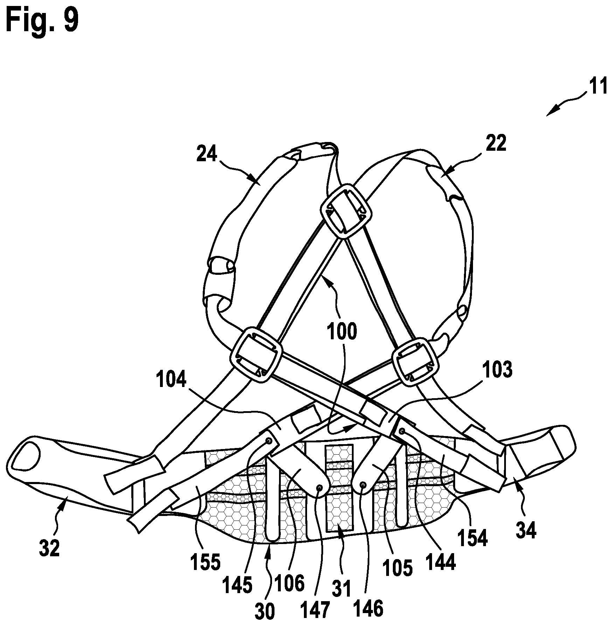

Figur 9 zeigt eine zweite Ausführungsform der erfindungsgemäßen Rückenorthese (11). Der Verlauf der Schultergurte (22, 24) und der Aufbau des Beckengurts (30) ist gleich wie bei der Ausführungsform aus Figur 1. In der vorliegenden Ausführungsform (11) sind jedoch das dritte Führungselement (103) und das vierte Führungselement (104) nicht direkt über das vierte Verbindungselement (144) und das fünfte Verbindungselement (145) mit der Bandage (30) verbunden, sondern das dritte Führungselement (103) ist mit einem fünften Führungselement (105) und das vierte Führungselement (104) mit einem sechsten Führungselement (106) gelenkig verbunden. Erst das fünfte Führungselement (105) und das sechste Führungselement (106) sind über das zusätzliche sechste Verbindungselement (146) und siebte Verbindungselement (147) mit dem Mittelabschnitt (31) des Beckengurts (30) gelenkig verbunden. Darüber hinaus sind in dieser Ausführungsform Krafteinleitungsgurte vorgesehen, wobei ein erster Krafteinleitungsgurt (154) das vierte Verbindungselement (144) mit dem rechten Seitenabschnitt (34) der Bandage (30) über einen Klettverschluss reversibel verbindet und ein zweiter Krafteinleitungsgurt (155) das fünfte Verbindungselement (145) mit dem linken Seitenabschnitt (32) des Beckengurts (30) über einen Klettverschluss reversibel verbindet.

In dieser Ausführungsform werden also durch das Führungsmodul (100) durch die zusätzlichen Verbindungselemente (105, 106) nicht anderthalb Rauten, sondern nahezu zwei Rauten gebildet.

Es ist erfindungsgemäß nicht ausgeschlossen das Führungsmodul (100) durch weitere Führungselemente und Verbindungselemente und somit weitere Rauten, beispielsweise um eine weitere Raute, weitere zwei Rauten, weitere drei Rauten, weitere vier Rauten, weitere fünf Rauten oder mehr zu ergänzen.

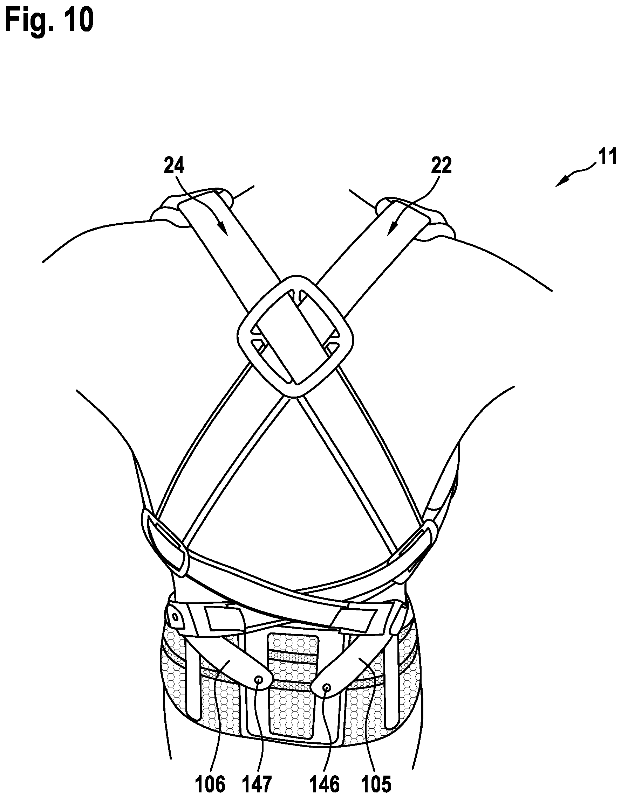

Figur 10 zeigt die Rückenorthese (11) aus Figur 9 in Rückansicht. Dabei sind deutlich im Vergleich zu Figur 4 die zusätzlichen Führungselemente (105, 106) und die zusätzlichen Verbindungselemente (146, 147) zu erkennen. Der Verlauf der beiden Schultergurte (22, 24) ist der gleiche wie in Figur 4, da die Schultergurte (22, 24) bereits an dem dritten beziehungsweise vierten Führungselement enden und an diesem reversibel und spannbar gekoppelt sind.

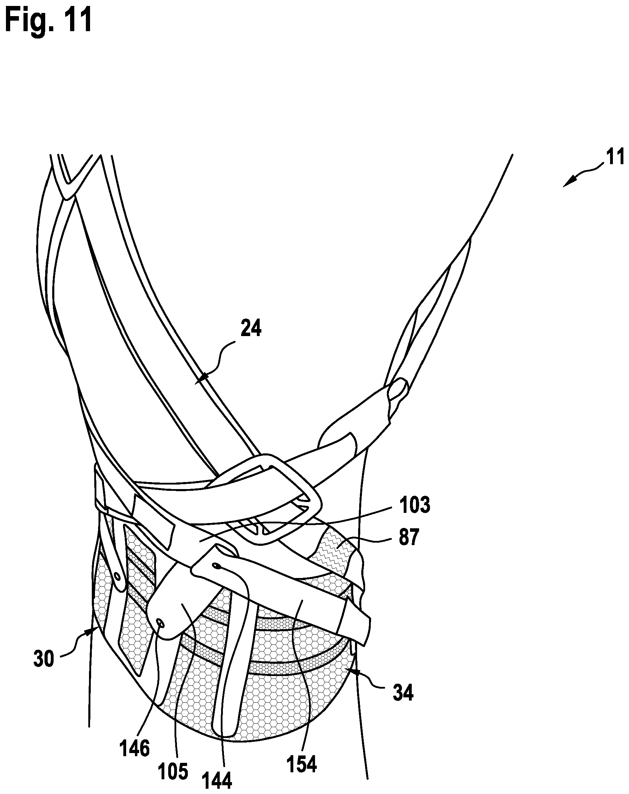

Figur 11 zeigt die Rückenorthese (11) von Figur 9 in der rechten Seitenansicht. Zu sehen ist die konstruktive Ausgestaltung, bei der das dritte Führungselement (103) über das vierte Verbindungselement (144) beweglich mit dem fünften Führungselement (105) gekoppelt ist, wobei das fünfte Führungselement (105) mit dem Beckengurt (30) beweglich über ein sechstes Verbindungselement (146) verbunden ist. Das vierte Verbindungselement (144) koppelt gleichzeitig auch das fünfte Führungselement (105) und das dritte Führungselement (103) mit dem rechten Krafteinleitungsgurt (154), der über einen Klettverschluss reversibel und spannbar mit dem rechten Seitenabschnitt (34) des Beckengurts (30) verbunden ist.

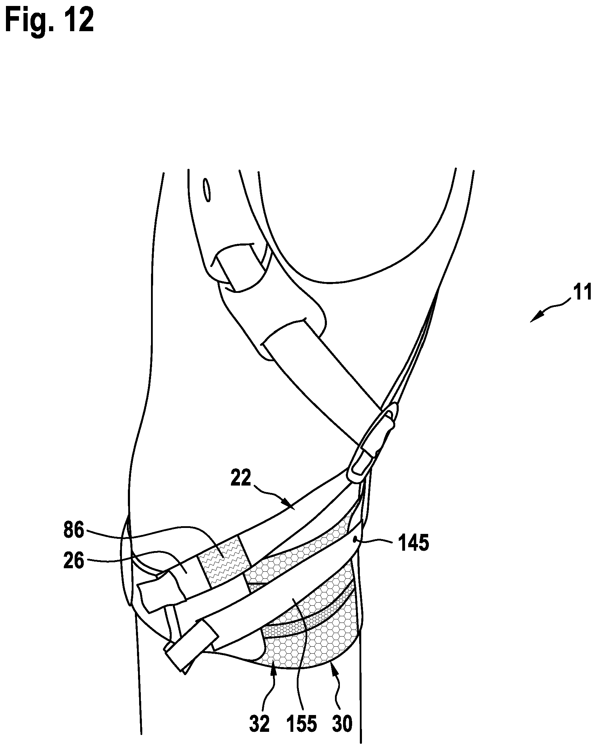

Der linke Schultergurt (24) hat hier noch zusätzlich einen elastischeren Gurtabschnitt (87). Figur 12 zeigt die Rückenorthese (11) aus Figur 9 in der linken Seitenansicht von schräg vorne. Zu sehen ist wie sowohl das erste Ende (26) des rechten Schultergurts (22), als auch der zweite Krafteinleitungsgurt (155), der am fünften Verbindungselement (145) beweglich befestigt ist, durch jeweils eine Klettverbindung am linken Seitenabschnitt (32) des Beckengurts (30) reversibel, positionierbar und somit spannbar befestigt sind.

Der rechte Schultergurt (22) hat hier noch zusätzlich einen elastischeren Gurtabschnitt (86).

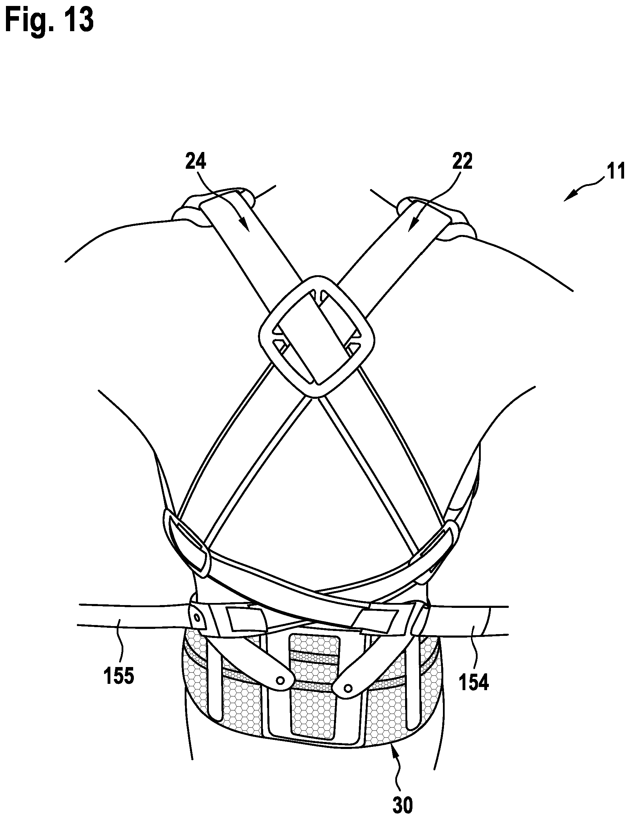

Figur 13 zeigt die Rückenorthese (11) von hinten, wie in Figur 10, wobei jedoch die beiden Krafteinleitungsgurte (154, 155) vom Beckengurt (30) gelöst sind. Die Krafteinleitungsgurte (154, 155) können so gespannt werden und dann in einem gespannten Zustand per Klettverschlüsse an dem Beckengurt (30) befestigt werden, was für den Träger in einfacher Weise eine Spannung des Gurtsystems aus den zwei Schultergurten (22, 24) erlaubt.

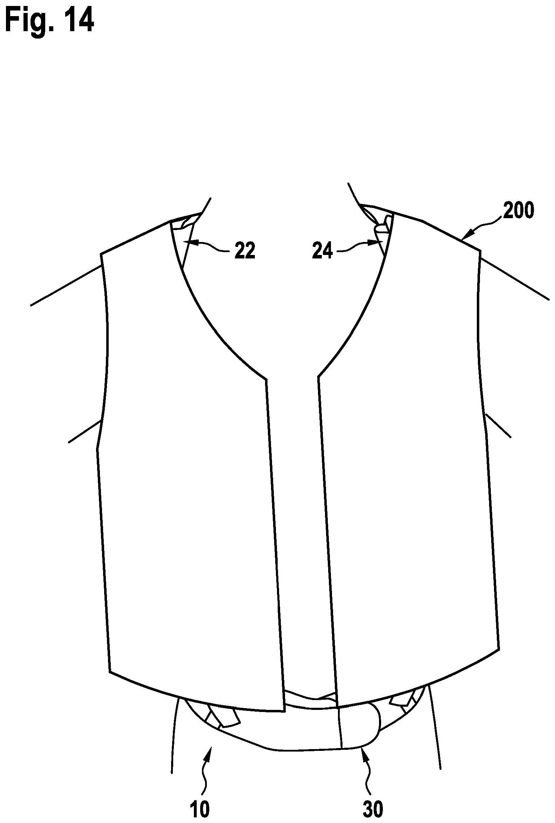

Figur 14 zeigt eine Jacke beziehungsweise Weste (200), die mit einer erfindungsgemäßen Rückenorthese (10) aufgerüstet ist. Dabei sind der rechte und linke Schultergurt (22, 24) und der Beckengurt (30) zumindest abschnittsweise fest mit der Weste (200) verbunden. Die Rückenorthese (10) kann so zusammen mit der Weste (200) direkt und unmittelbar angelegt werden. Eine solche Weste eignet sich beispielsweise als Arbeitsschutzkleidung.

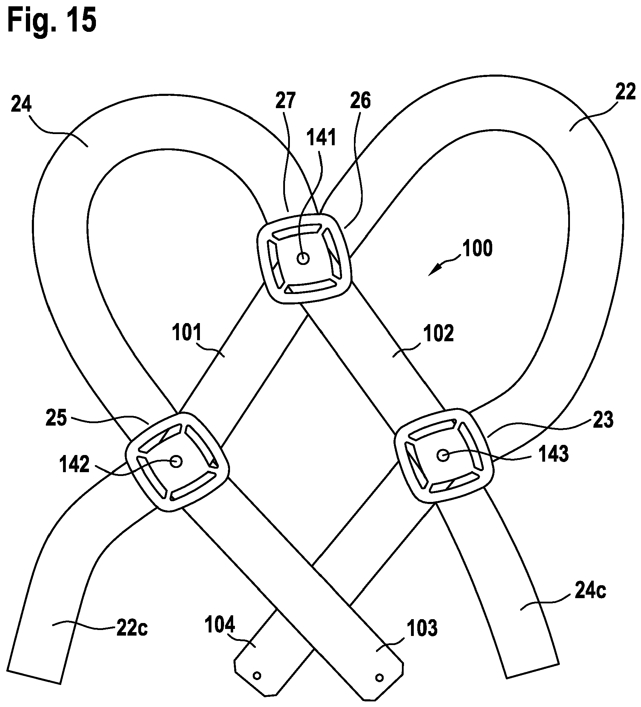

Figur 15 zeigt das Grundprinzip einer alternativen Ausführungsform der erfindungsgemäßen Rückenorthese. Hier verlaufen die Schultergurte (22,24) nicht auf dem Führungsmodul (100), sondern die Schultergurte (22,24) enden oben am Führungsmodul (100) und sind dort mit diesem befestigt. In dieser Ausführungsform bildet das Führungsmodul (100) ein Zwischenstück am Rücken zwischen Schultergurten (22,24) und Beckengurt. Somit ist das Führungsmodul (100) beziehungsweise sind die Führungselemente (101, 102, 103, 104) Teil des Gurtverlaufs.

In dieser Ausführungsform kann das zweite Ende (25) des linken Schultergurts (24) im Bereich des zweiten Verbindungselements (142) mit dem dritten Führungselement (103) verbunden sein und das zweite Ende (23) des rechten Schultergurts (22) im Bereich des dritten Verbindungselements (143) mit dem vierten Führungselement (104) verbunden sein. In dieser Ausführungsform kann auch das erste Ende (27) des linken Schultergurts (24) im Bereich des ersten Verbindungselements (141) mit dem zweiten Führungselement (102) verbunden sein und das erste Ende (26) des rechten Schultergurts (22) im Bereich des ersten Verbindungselements (141) mit dem ersten Führungselement (101) verbunden sein. Die jeweiligen Verbindungen können natürlich reversibel gestaltet sein.

Zusätzlich kann vorgesehen sein, dass ein erster zusätzlicher Gurt (24c) im Bereich des dritten Verbindungselements (143) mit dem zweiten Führungselement (102) verbunden ist und zum rechten Seitenabschnitt des Beckengurtes (nicht gezeigt) führt und mit diesem verbunden ist und ein zweiter zusätzlicher Gurt (22c) im Bereich des zweiten Verbindungselements (142) mit dem ersten Führungselement (101) verbunden ist und zum linken Seitenabschnitt des Beckengurtes (nicht gezeigt) führt und mit diesem verbunden ist. Die jeweiligen Verbindungen können natürlich reversibel gestaltet sein.

## Patentansprüche

1. Rückenorthese (10,11), enthaltend:
- einen Beckengurt (30) zum Anlegen an das Becken eines Trägers, mit einem lumbal/sakral positionierbarem Mittelabschnitt (31) und davon jeweils seitlich abgehenden linkem Seitenabschnitt (32) mit linkem Beckengurtende (33) und rechtem Seitenabschnitt (34) mit rechtem Beckengurtende (35), wobei die Beckengurtenden (33, 35) zum Anlegen miteinander unter Spannung verbindbar sind,
- einen rechten Schultergurt (22) zum Anlegen über die rechte Schulter des Trägers, der über sein erstes Ende (26) mit dem linken Seitenabschnitt (32) des Beckengurts (30) verbunden ist, und
- einen linken Schultergurt (24) zum Anlegen über die linke Schulter des Trägers, der über sein erstes Ende (27) mit dem rechten Seitenabschnitt (34) des Beckengurts (30) verbunden ist,
wobei sich rechter Schultergurt (22) und linker Schultergurt (24) in einem im angelegten Zustand dorsalen mittigen oberen Kreuzungspunkt (41), einem im angelegten Zustand dorsalen mittigen unteren Kreuzungspunkt (43) und in zwei im angelegten Zustand jeweils seitlichen Kreuzungspunkten (42,44) kreuzen,
**dadurch gekennzeichnet, dass**
die Rückenorthese ein den Schultergurten (22,24) zugeordnetes Führungsmodul (100) aufweist, wobei das Führungsmodul (100) umfasst:
- ein erstes Führungselement (101),
- ein zweites Führungselement (102)
- ein drittes Führungselement (103)
- ein viertes Führungselement (104)
- ein erstes Verbindungselement (141)
- ein zweites Verbindungselement (142)
- ein drittes Verbindungselement (143)
- ein viertes Verbindungselement (144)
- ein fünftes Verbindungselement (145),
wobei das erste Verbindungselement (141) am dorsalen mittigen oberen Kreuzungspunkt (41) positioniert ist und das erste Führungselement (101) und das zweite Führungselement (102) als Gelenk miteinander verbindet, wobei das zweite Verbindungselement (142) am ersten seitlichen Kreuzungspunkt (42) positioniert ist und das erste Führungselement (101) und das dritte Führungselement (103) als Gelenk miteinander verbindet, wobei das dritte Verbindungselement (143) am zweiten seitlichen Kreuzungspunkt (44) positioniert ist und das zweite Führungselement (102) und das vierte Führungselement (104) als Gelenk miteinander verbindet, wobei sich das dritte Führungselement (103) und das vierte Führungselement (104) im Bereich des dorsalen mittigen unteren Kreuzungspunkts (43) überkreuzen,
wobei der rechte Schultergurt (22) über sein zweites Ende (23) und der linke Schultergurt (24) über sein zweites Ende (25) mit dem Führungsmodul (100) verbunden sind, insbesondere der rechte Schultergurt (22) über sein zweites Ende (23) mit dem vierten Führungselement (104) reversibel verbunden ist und wobei der linke Schultergurt (24) über sein zweites Ende (25) mit dem dritten Führungselement (103) reversibel verbunden ist.

2. Rückenorthese (10) nach Anspruch 1, **dadurch gekennzeichnet, dass** das dritte Führungselement (103) über das vierte Verbindungselement (144) als Gelenk und das vierte Führungselement (104) über das fünfte Verbindungselement (145) als Gelenk direkt mit dem Mittelabschnitt (31) des Beckengurts (30) verbunden sind.

3. Rückenorthese (11) nach Anspruch 1, **dadurch gekennzeichnet, dass** vierte Verbindungselement (144) das dritte Führungselement (103) und ein fünftes Führungselement (105) als Gelenk miteinander verbindet, wobei das fünfte Verbindungselement (145) das vierte Führungselement (104) und ein sechstes Führungselement (106) als Gelenk miteinander verbindet, wobei das fünfte Führungselement (105) über ein sechstes Verbindungselement (146) als Gelenk und das sechste Führungselement (106) über ein siebtes Verbindungselement (147) als Gelenk mit dem Mittelabschnitt (31) des Beckengurts (30) verbunden sind.

4. Rückenorthese nach einem der vorstehenden Ansprüche, **dadurch gekennzeichnet, dass** das erste Verbindungselement (141), das zweite Verbindungselement (142) und/oder das dritte Verbindungselement (143) Durchführungsöffnungen (151, 152, 153) aufweisen, durch die der rechte Schultergurt (22) und der linke Schultergurt (24) verschieblich geführt sind.

5. Rückenorthese nach Anspruch 4, **dadurch gekennzeichnet, dass** das erste Verbindungselement (141), das zweite Verbindungselement (142) und das dritte Verbindungselement (143) jeweils eine erste Durchführungsöffnung (151a, 152a, 153a) aufweisen, durch die der rechte Schultergurt (22) verschieblich geführt ist und jeweils eine zweite Durchführungsöffnung (151b, 152b, 153b) aufweisen, durch die der linke Schultergurt (24) verschieblich geführt ist.

6. Rückenorthese nach einem der vorstehenden Ansprüche, **dadurch gekennzeichnet, dass** die Führungselemente (101,102,103,104,105,106) aus einem flexiblen und in Zugrichtung im wesentlichen unelastischen Material, beispielsweise Kunststoff, Leder oder Metall, insbesondere Federbandstahl, gebildet sind.

7. Rückenorthese nach einem der vorstehenden Ansprüche, wobei jeweils zwischen dem ersten Ende des Schultergurts (26, 27) und dem seitlichen Kreuzungspunkt (42, 44) ein elastischer Gurtabschnitt (86,87) ausgebildet ist, dessen Elastizität gleich oder höher ist als diejenige des Schultergurts (22, 24) selbst.

8. Rückenorthese nach einem der vorstehenden Ansprüche, wobei die ersten Enden (26,27) der Schultergurte (22,24) reversibel, insbesondere über einen Klettverschluss mit dem Beckengurt (30) verbunden sind.

9. Rückenorthese nach einem der vorstehenden Ansprüche, wobei die zweiten Enden (23,25) der Schultergurte (22,24) an den zugeordneten Führungselementen (104,103) variabel einstellbar sind oder das vierte und das dritte Führungselement (104,103) jeweils mindestens ein Umlenkelement (164,163) aufweisen, durch das jeweils der zugeordnete Schultergurt (22,24) hindurchgeführt ist und das zweite Ende (23,25) reversibel und variabel am Schultergurt (22,24) befestigt werden kann.

10. Rückenorthese nach einem der Ansprüche 3 bis 9, wobei an dem vierten Verbindungselement (144) und an dem fünften Verbindungselement (145) jeweils ein gelenkig verbundener Krafteinleitungsgurt (154, 155) befestigt ist, wobei die Krafteinleitungsgurte (154, 155) reversibel mit dem Beckengurt verbunden sind.

11. Rückenorthese nach einem der vorstehenden Ansprüche, wobei die Schultergurte (22,24) nicht auf dem Führungsmodul (100) verlaufen, sondern wobei die Schultergurte (22,24) oben am Führungsmodul (100) enden und dort mit diesem befestigt sind.

12. Rückenorthese nach einem der vorstehenden Ansprüche, wobei die Schultergurte und/oder die Krafteinleitungsgurte durch ein Seilzugsystem gebildet werden.

13. Bekleidungsstück, enthaltend eine dehnbare Weste oder Jacke (200) und ein Rückenorthese charakterisiert in einem der vorstehenden Ansprüche, wobei Schultergurte (22, 24) und Beckengurt (30) zumindest abschnittsweise auf einer Weste oder Jacke (200) aufgerüstet und mit dieser fest verbunden sind.

## Claims

1. A back orthosis (10,11) containing:
- a pelvic belt (30) for applying to the pelvis of a wearer, having a lumbar/sacral positionable middle section (31) and, extending laterally therefrom in each case, a left side section (32) with a left pelvic belt end (33) and a right side section (34) with a right pelvic belt end (35), wherein the pelvic belt ends (33, 35) are connectable to one another under tension for applying,
- a right shoulder belt (22) for applying over the right shoulder of the wearer, connected by its first end (26) to the left side section (32) of the pelvic belt (30), and
- a left shoulder belt (24) for applying over the left shoulder of the wearer, connected by its first end (27) to the right side section (34) of the pelvic belt (30),
wherein the right shoulder belt (22) and the left shoulder belt (24) cross at a dorsal mid-upper crossing point (41) in the applied state, a dorsal mid-lower crossing point (43) in the applied state and at two lateral crossing points (42, 44) each in the applied state,
**characterised in that**
the back orthosis has a guiding module (100) associated with the shoulder belts (22, 24), wherein the guiding module (100) comprises
- a first guiding element (101),
- a second guiding element (102)
- a third guiding element (103)
- a fourth guiding element (104)
- a first connecting element (141)
- a second connecting element (142)
- a third connecting element (143)
- a fourth connecting element (144)
- a fifth connecting element (145),
wherein the first connecting element (141) is positioned at the dorsal mid-upper crossing point (41) and connects the first guiding element (101) and the second guiding element (102) to one another as a joint, wherein the second connecting element (142) is positioned at the first lateral crossing point (42) and connects the first guiding element (101) and the third guiding element (103) to one another as a j oint, wherein the third connecting element (143) is positioned at the second lateral crossing point (44) and connects the second guiding element (102) and the fourth guiding element (104) to one another as a joint, wherein the third guiding element (103) and the fourth guiding element (104) cross over in the region of the dorsal mid-lower crossing point (43),
wherein the right shoulder belt (22) is connected to the guiding module (100) via its second end (23) and the left shoulder belt (24) is connected to the guiding module (100) via its second end (25), in particular the right shoulder belt (22) is reversibly connected to the fourth guiding element (104) via its second end (23) and wherein the left shoulder belt (24) is reversibly connected to the third guiding element (103) via its second end (25).

2. The back orthosis (10) of claim 1, **characterised in that** the third guiding element (103) is connected directly to the middle section (31) of the pelvic belt (30) via the fourth connecting element (144) as a joint and the fourth guiding element (104) is connected directly to the middle section (31) of the pelvic belt (30) via the fifth connecting element (145) as a joint.

3. The back orthosis (11) of claim 1, **characterized in that** the fourth connecting element (144) connects the third guiding element (103) and a fifth guiding element (105) to one another as a joint, wherein the fifth connecting element (145) connects the fourth guiding element (104) and a sixth guiding element (106) to one another as a joint, wherein the fifth guiding element (105) is connected to the middle section (31) of the pelvic belt (30) via a sixth connecting element (146) as a joint and the sixth guiding element (106) is connected to the middle section (31) of the pelvic belt (30) via a seventh connecting element (147) as a joint.

4. The back orthosis of one of the preceding claims, **characterized in that** the first connecting element (141), the second connecting element (142) and/or the third connecting element (143) have pass-through openings (151, 152, 153) through which the right shoulder belt (22) and the left shoulder belt (24) are slidably guided.

5. The back orthosis of claim 4, **characterized in that** the first connecting element (141), the second connecting element (142) and the third connecting element (143) each have a first pass-through opening (151a, 152a, 153a) through which the right shoulder belt (22) is slidably guided and each have a second pass-through opening (151b, 152b, 153b) through which the left shoulder belt (24) is slidably guided.

6. The back orthosis of one of the preceding claims, **characterised in that** the guiding elements (101, 102, 103, 104, 105, 106) are formed from a flexible material which is substantially inelastic in the direction of tension, for example plastic, leather or metal, in particular spring steel strip.

7. The back orthosis of one of the preceding claims, wherein an elastic belt section (86, 87) is formed between the first end of the shoulder belt (26, 27) and the lateral crossing point (42, 44), respectively, the elasticity of which is equal to or higher than that of the shoulder belt (22, 24) itself.

8. The back orthosis of one of the preceding claims, wherein the first ends (26, 27) of the shoulder belts (22, 24) are reversibly connected to the pelvic belt (30), in particular via a Velcro fastener.

9. The back orthosis of one of the preceding claims, wherein the second ends (23, 25) of the shoulder belts (22, 24) are variably adjustable on the associated guiding elements (104, 103) or the fourth and the third guiding element (104, 103) each have at least one deflecting element (164, 163) through which the associated shoulder belt (22, 24) is guided through respectively and the second end (23, 25) can be reversibly and variably fastened to the shoulder belt (22, 24).

10. The back orthosis of any one of claims 3 to 9, wherein a hingedly connected force introducing belt (154, 155) is fastened to the fourth connecting element (144) and to the fifth connecting element (145), respectively, wherein the force introducing belts (154, 155) are reversibly connected to the pelvic belt.

11. The back orthosis of any one of the preceding claims, wherein the shoulder belts (22, 24) do not run on the guiding module (100), but wherein the shoulder belts (22, 24) end at the top of the guiding module (100) and are fastened thereto.

12. The back orthosis of one of the preceding claims, wherein the shoulder belts and/or the force introducing belts are formed by a cable pull system.

13. A clothing item containing a stretchable waistcoat or jacket (200) and a back orthosis **characterised in** one of the preceding claims, wherein shoulder belts (22, 24) and pelvic belt (30) are at least sectionally rigged on a waistcoat or jacket (200) and fixedly connected thereto.

## Revendications

1. Une orthèse dorsale (10, 11) comportant :
- une ceinture pelvienne (30) pour appliquer sur le pelvis d'un porteur, ayant une section médiane (31) positionnable au niveau lombaire/sacré et, s'étendant latéralement à partir de celle-ci dans chacun une section latérale gauche (32) avec une extrémité gauche de ceinture pelvienne (33) et une section latérale droite (34) avec une extrémité droite de ceinture pelvienne (35), dans lequel les extrémités de ceinture pelvienne (33, 35) sont connectables l'une à l'autre sous tension pour appliquer,
- une ceinture d'épaule droite (22) pour appliquer sur l'épaule droite du porteur, connectée par sa première extrémité (26) à la section latérale gauche (32) de la ceinture pelvienne (30), et
- une ceinture d'épaule gauche (24) pour appliquer sur l'épaule gauche du porteur, connectée par sa première extrémité (27) à la section latérale droite (34) de la ceinture pelvienne (30),
dans lequel la ceinture d'épaule droite (22) et la ceinture d'épaule gauche (24) se croisent en un point de croisement dorsal médian supérieur (41) à l'état appliqué, un point de croisement dorsal médian inférieur (43) à l'état appliqué et à deux points de croisement latéraux (42, 44) chacun à l'état appliqué,
**caractérisé en ce que**
l'orthèse dorsale a un module de guidage (100) associé aux ceintures d'épaule (22, 24), dans lequel le module de guidage (100) comprend :
- un premier élément de guidage (101),
- un deuxième élément de guidage (102)
- un troisième élément de guidage (103)
- un quatrième élément de guidage (104)
- un premier élément de connexion (141)
- un deuxième élément de connexion (142)
- un troisième élément de connexion (143)
- un quatrième élément de connexion (144)
- un cinquième élément de connexion (145),
dans lequel le premier élément de connexion (141) est positionné au point de croisement dorsal médian supérieur (41) et connecte le premier élément de guidage (101) et le deuxième élément de guidage (102) l'un à l'autre en tant qu'articulation, dans lequel le deuxième élément de connexion (142) est positionné au premier point de croisement latéral (42) et connecte le premier élément de guidage (101) et le troisième élément de guidage (103) l'un à l'autre en tant qu'articulation, dans lequel le troisième élément de connexion (143) est positionné au deuxième point de croisement latéral (44) et connecte le deuxième élément de guidage (102) et le quatrième élément de guidage (104) l'un à l'autre en tant qu'articulation, dans lequel le troisième élément de guidage (103) et le quatrième élément de guidage (104) se croisent dans la région du point de croisement dorsal médian inférieur (43),
dans lequel la ceinture d'épaule droite (22) est connectée au module de guidage (100) par sa deuxième extrémité (23) et la ceinture d'épaule gauche (24) est connectée au module de guidage (100) par sa deuxième extrémité (25), en particulier la ceinture d'épaule droite (22) est connectée réversiblement au quatrième élément de guidage (104) par sa deuxième extrémité (23) et dans lequel la ceinture d'épaule gauche (24) est connectée réversiblement au troisième élément de guidage (103) par sa deuxième extrémité (25).

2. L'orthèse dorsale (10) selon la revendication 1, **caractérisée en ce que** le troisième élément de guidage (103) est connecté directement à la section médiane (31) de la ceinture pelvienne (30) par le quatrième élément de connexion (144) en tant qu'articulation et le quatrième élément de guidage (104) est connecté directement à la section médiane (31) de la ceinture pelvienne (30) par le cinquième élément de connexion (145) en tant qu'articulation.

3. L'orthèse dorsale (11) selon la revendication 1, **caractérisé en ce que** le quatrième élément de connexion (144) connecte le troisième élément de guidage (103) et un cinquième élément de guidage (105) l'un à l'autre en tant qu'articulation, dans lequel le cinquième élément de connexion (145) connecte le quatrième élément de guidage (104) et un sixième élément de guidage (106) l'un à l'autre en tant qu'articulation, dans lequel le cinquième élément de guidage (105) est connecté à la section médiane (31) de la ceinture pelvienne (30) par un sixième élément de connexion (146) en tant qu'articulation et dans lequel le sixième élément de guidage (106) est connecté à la section médiane (31) de la ceinture pelvienne (30) par un septième élément de connexion (147) en tant qu'articulation.

4. L'orthèse dorsale selon l'une des revendications précédentes, **caractérisé en ce que** le premier élément de connexion (141), le deuxième élément de connexion (142) et/ou le troisième élément de connexion (143) ont des ouvertures de passage (151, 152, 153) à travers lesquelles la ceinture d'épaule droite (22) et la ceinture d'épaule gauche (24) sont guidées de manière coulissante.

5. L'orthèse dorsale selon la revendication 4, **caractérisé en ce que** le premier élément de connexion (141), le deuxième élément de connexion (142) et le troisième élément de connexion (143) ont chacun une première ouverture de passage (151a, 152a, 153a) à travers laquelle la ceinture d'épaule droite (22) est guidée de manière coulissante, et ont chacun une deuxième ouverture de passage (151b, 152b, 153b) à travers laquelle la ceinture d'épaule gauche (24) est guidée de manière coulissante.

6. L'orthèse dorsale selon l'une des revendications précédentes, **caractérisée en ce que** les éléments de guidage (101, 102, 103, 104, 105, 106) sont formés à partir d'un matériau flexible qui est substantiellement inélastique dans le sens de la tension, par exemple du plastique, du cuir ou du métal, en particulier une bande d'acier à ressort.

7. L'orthèse dorsale selon l'une des revendications précédentes, dans lequel une section de ceinture élastique (86, 87) est formée entre la première extrémité de la ceinture d'épaule (26, 27) et le point de croisement latéral (42, 44), respectivement, dont l'élasticité est égale ou supérieure à celle de la ceinture d'épaule (22, 24) elle-même.

8. L'orthèse dorsale selon l'une des revendications précédentes, dans lequel les premières extrémités (26, 27) des ceintures d'épaule (22, 24) sont connectées réversiblement à la ceinture pelvienne (30), en particulier par une fermeture Velcro.

9. L'orthèse dorsale selon l'une des revendications précédentes, dans lequel les secondes extrémités (23, 25) des ceintures d'épaule (22, 24) sont réglables variablement sur les éléments de guidage associés (104, 103) ou le quatrième et le troisième élément de guidage (104, 103) ont chacun au moins un élément de déviation (164, 163) à travers lequel la ceinture d'épaule associée (22, 24) est guidée et la deuxième extrémité (23, 25) peut être fixée réversiblement et variablement à la ceinture d'épaule (22, 24).

10. L'orthèse dorsale selon l'une quelconque des revendications 3 à 9, dans lequel une ceinture d'introduction de force connectée de manière articulée (154, 155) est fixée respectivement au quatrième élément de connexion (144) et au cinquième élément de connexion (145), dans lequel les ceintures d'introduction de force (154, 155) ont connecté réversiblement à la ceinture pelvienne.

11. L'orthèse dorsale selon l'une quelconque des revendications précédentes, dans lequel les ceintures d'épaule (22, 24) ne passent pas sur le module de guidage (100), mais dans lequel les ceintures d'épaule (22, 24) se terminent au sommet du module de guidage (100) et y sont fixées.

12. L'orthèse dorsale selon l'une des revendications précédentes, dans lequel les ceintures d'épaule et/ou les ceintures d'introduction de force sont formées par un système de traction par câble.

13. Un vêtement contenant un gilet ou une veste (200) extensible et une orthèse dorsale caractérisée dans l'une des revendications précédentes, dans lequel les ceintures d'épaule (22, 24) et la ceinture pelvienne (30) sont au moins partiellement fixées sur un gilet ou une veste (200) et connectées de manière fixe à celui-ci.
